## Europäisches Patentamt

(19) **European Patent Office**

**Office européen des brevets**

(11) Numéro de publication: **0 070 048**

**B1**

(12) # FASCICULE DE BREVET EUROPÉEN

(45) Date de publication du fascicule du brevet:
31.05.89

(51) Int. Cl.⁴: **C 07 C 101/72,** C 07 C 101/24, C 07 C 101/30, C 07 C 129/08, C 07 C 127/15

(21) Numéro de dépôt: **82200649.0**

(22) Date de dépôt: **08.10.79**

(54) **Dérivés de tyrosine.**

(30) Priorité: **19.10.78 FR 7829797**

(43) Date de publication de la demande:
**19.01.83 Bulletin 83/3**

(45) Mention de la délivrance du brevet:
**31.05.89 Bulletin 89/22**

(84) Etats contractants désignés:
**AT BE CH DE GB IT LU NL SE**

(56) Documents cité:
**BE-A-458 709**
**FR-A-2 034 543**

**H.JANISTYN: "Handbuch der Kosmetika und Riechstoffe", vol. 1, 2ème édition, 1969, Hüthig Verlag Heidelberg, pages 1117-1119.**

**Le dossier contient des informations techniques présentées postérieurement au dépôt de la demande et ne figurant pas dans le présent fascicule.**

(73) Titulaire: **LABORATOIRES SEROBIOLOGIQUES S.A., F-54 420 Pulnoy (FR)**

(72) Inventeur: **Pauly, Marc, 10, rue Joffre, F-57170 Château- Salins (FR)**

(74) Mandataire: **Portal, Gérard, Cabinet Beau de Loménie 55, rue d'Amsterdam, F-75008 Paris (FR)**

## Description

La présente invention a essentiellement pour objet de nouveaux dérivés de tyrosine et un procédé chimique de préparation de ces nouveaux dérivés de tyrosine.

Ces nouveaux dérivés de tyrosine peuvent être incorporés dans une composition, utilisable notamment comme produit cosmétique destinée à être appliquée topiquement sur l'épiderme pour favoriser la photo-pigmentation de celui-ci et/ou sa photo-protection par exposition au rayonnement solaire ou ultraviolet, telle que décrite et revendiquée dans la demande de brevet européen 0 010 483 inclus ici pour référence.

On connaît déjà des compositions favorisant le bronzage de la peau et/ou sa photo-protection. Par exemple, on connaît des préparations dites anti-solaires qui permettent l'exposition au soleil, avec bronzage, tout en protégeant la peau des rayonnenents UV B, réputés érythématogènes et dangereux, qui utilisent des substances écran ou filtres solaires préparées par synthèse telles que les salicylates, anthranilates, cinnamates ou esters para-amino benzoïques ou d'origine naturelle obtenues par extraction à partir de végétaux, par exemple huiles végétales, sésame, camomille.

D'autres préparations connues utilisent des photo-sensibilisants appliqués par voie interne ou topiqué, préparés par synthèse tels que les furocoumarinés ou le 5 ou 8-méthoxypsolarène (M. O. P) ou d'origine naturelle tels que l'essence de bergamote, la vitamine A ou F.

On connaît également d'autres produits dits bronzants ou tannants sans soleil qui utilisent des agents chimiques tels que dihydroxyacétone, érythrulose, glycérose. De même, d'autres préparations connues utilisent des produits colorants ou teintés qui procurent un teint hâlé ou bronzé.

Cependant, toutes ces préparations ou compositions connues permettant un bronzage de la peau avec ou sans soleil utilisent des agents étrangers à la peau et présentent des inconvénients très importants et bien connus. En effet, ces agents étrangers sont rarement substantifs. D'autre part, ils sont plus ou moins bien tolérés. En outre, ils doivent être utilisés en applications renouvelées et fréquentes compte tenu de la difficulté de les faire pénétrer dans les couches externes de l'épiderme.

Certaines de ces préparations connues sont même dangereuses, en particulier les préparations à base de photo-sensibilisants et doivent donc être utilisées avec précautions.

Par ailleurs, on connaît aussi à partir du brevets belge n° 458 709 une composition comportant principalement l'emploi d'une tyrosinase en vue de réaliser un effet bronzant par action oxydante sur la tyrosine présente dans la peau ou s'y trouvant par l'application préalable de substances la contenant. Selon ce document, on utilise principalement la tyrosinase alors que la tyrosine est employée tout à fait accessoirement. En outre, la tyrosinase utilisée selon ce document présente un inconvénient majeur à savoir qu'elle ne peut être utilisée dans les préparations cosmétiques courantes en raison de son instabilité dans sa forme dissoute. D'autre part, ce document ne précise pas les conditions d'inhibition de cette enzyme.

Enfin, selon ce document, la tyrosine est employée telle quelle, c'est-à-dire sous une forme libre et en solution aqueuse de sorte que ce document ne permet pas de résoudre les inconvénients précités. Il n'arrive dans le meilleur des cas qu'à fournir une pigmentaton superficielle de l'épiderme et la composition selon ce document doit donc être utilisée en application renouvelée et fréquente compte tenu de la difficulté de la faire pénétrer dans les couches externes de l'épiderme.

D'autre part, un ouvrage de JANYSTIN HANDBUCH DER KOSMETICA UND RIECHSTOFFE, volume 1/1969, pages 1117 - 1119 cite sur des bases théoriques un certain nombre d'amino-acides dont on indique notamment simplement que la tyrosine "doit" procurer un brunissement de la peau, une telle mention théorique ne pouvant en aucun cas être utilisable en pratique pour un homme du métier.

L'art antérieur n'a donc pas fourni de solution capable de rémédier aux inconvénients précités.

La présente invention a donc pour but de remédier aux inconvénients précités des préparations ou compositions bronzantes connues en fournissant de nouveaux dérivés permettant de réaliser une photo-pigmentation de l'épiderme et/ou sa photo-protection par exposition aux rayonnements solaires ou ultraviolets, qui soient bien tolérés par l'épiderme et évitent l'apparition d'érythèmes ou "coups de soleil" douloureux et pouvant avoir des conséquences plus ou moins graves.

La solution à ce nouveau problème technique doit être de préférence de conception particulièrement simple.

Cette solution consiste, selon la présente invention, en de nouveaux dérivés de tyrosine, caractérisés en ce qu'il s'agit du sel complexe de L-tyrosine avec au moins un acide aminé basique choisi parmi l'arginine, l'ornithine, la citrulline, la lysine ou l'hydroxylysine.

Selon un mode de réalisation préféré, il s'agit du sel complexe de L-tyrosine et de L-arginine, de préférence constitué par 47 % en poids de L-tyrosine et 53 % en poids de L-arginine.

Ainsi, avec l'emploi de ces nouveaux dérivés de tyrosine, on n'utilise que des substances homologues ou analogues à celles existant normalement dans l'épiderme et qui jouent directement ou indirectement un rôle dans la mélanogénèse. Une composition cosmétique, ou dermo-corrective, préparée avec de tels composés est donc parfaitement tolérée par le sujet.

D'autres buts, caractéristiques et avantages de la présente invention apparaîtront plus clairement à la lumière des exemples suivants donnés simplement à titre d'illustration de la préparation et de l'emploi des nouveaux dérivés de tyrosine dans des compositions cosmétiques et dermo-correctives et qui ne sauraient donc en aucune façon limiter la portée de la présente invention.

Dans les exemples, toutes les proportions sont données en pourcentage en poids.

On prépare un complexe de L-tyrosine-L-arginine base qui est parfaitement soluble et stable et dont on peut régler le pH sur la neutralité ou sur le pH émidermique en faisant varier les proportions de chacun des constituants.

Par exemple, ce complexe est formé de 47 % de L-tyrosine et 53 % de L-arginine base.

Ce complexe est ensuite traité et utilisé comme décrit dans la demande de brevet européen N° 0 010 483 à l'exemple 1, pour préparer une composition utilisable notamment en cosmétique et dans les produits solaires.

On a également préparé par mélange d'autres complexes L-tyrosine-L-arginine dans des proportions en poids différentes, ainsi que des complexes L-tyrosine-L-ornithine; L-citrulline ou L-lysine pour la réalisation des compositions décrites et revendiquées dans la demande de brevet européen N° 0 010 483.

**Revendications**

1. Dérivés de tyrosine, caractérisés en ce qu'il s'agit du sel complexe de L-tyrosine avec au moins un acide aminé basique choisi parmi l'arginine, l'ornithine, la citrulline, la lysine, ou l'hydroxylysine.

2. Dérivés de tyrosine selon la revendication 1, caractérisé en ce qu'il s'agit du sel complexe de L-tyrosine et de L-arginine, de préférence constitué par 47 % de L-tyrosine et 53 % de L-arginine, en poids.

**Patentansprüche**

1. Tyrosinderivate, dadurch gekennzeichnet, daß es sich um das Komplexsalz von L-Tyrosin mit mindestens einer der basischen Aminosäuren Arginin, Ornithin, Zitrullin, Lysin oder Hydroxylysin handelt.

2. Tyrosinderivate nach Anspruch 1, dadurch gekennzeichnet, daß es sich um das Komplexsalz von L-Tyrosin und L-Arginin handelt, vorzugsweise bestehend aus 47 Gew.-% L-Tyrosin und 53 Gew.-% L-Arginin.

**Claims**

1. Tyrosine derivatives, characterized in that they consist in the complex salt of L-tyrosine with at least one basic amino acid seiected from arginine, ornithine, citrulline, lysine, or hydroxylysine.

2. Tyrosine derivatives, according to claim 1, characterized in that they consist in the compiex salt of L-tyrosine and of L-arginine, preferably constituted by 47 % by weight of L-tyrosine and 53 % by weight of L-arginine.